# EUROPEAN PATENT SPECIFICATION

(11) **EP 3 689 304 B1**
(45) Date of publication and mention of the grant of the patent: **17.01.2024**
(21) Application number: 19305119.0
(22) Date of filing: 31.01.2019
(51) Int. Cl.: A61F 9/06, A61F 9/02, G02C 11/00, A41D 13/11, A61B 5/00

(54) **PROTECTIVE EYEWEAR APPARATUS**
SCHUTZBRILLENVORRICHTUNG
APPAREIL À LUNETTES DE PROTECTION

(43) Date of publication of application: 05.08.2020
(73) Proprietor: Bollé Protection, 69100 Villeurbanne (FR)
(72) Inventor: GAFSOU, David, 69100 Villeurbanne (FR); DE SEREYS, Ludovic, 69100 Villeurbanne (FR); MEUNIER, Rubina, 69100 Villeurbanne (FR); GUILLOBEZ, Damien, 69100 Villeurbanne (FR)
(74) Representative: Regimbeau

(56) References cited:
- WO-A1-2007/083900
- US-A- 3 533 686
- US-A1- 2008 218 684
- US-A1- 2018 224 673
- US-B1- 6 192 523

## Description

### FIELD OF INVENTION

The present invention relates to protective eyewear, and more particularly to a multi-component eyewear apparatus attachable to a garment for use in a controlled environment.

### BACKGROUND

In any industrial or commercial in which a controlled environment is present, any individual entering or operating within that environment must take precautions to protect herself from the environmental hazards present. Similarly, the person must ensure the environment maintains stability and sterility by preventing contaminants the individual may be carrying from entering the controlled environment. In many cases, individuals entering or operating in such controlled environments wear protective eyewear, breathing masks, or an entire suit.

In certain environments, eyewear and breathing masks may be integrated not only with each other, but also with a protective garment, such as a hood or body-suit designed to protect the individual from the environment and protect the environment from the individual. Traditional integrated eyewear, however, may be costly, inefficient, and difficult to dispose of or clean. Additionally, traditional integrated safety equipment does not individually monitor or notify an individual of its use-time, expiration or other usage details. Document WO 2007/083900 teaches such a protective eyewear apparatus. Document US 2008/218684 teaches a pair of glasses comprising an RFID tag.

### SUMMARY

The invention is defined by the independent claims.

Embodiments of the present invention provide a protective eyewear apparatus for use in controlled environments. The protective eyewear may include a multi-component apparatus that can be easily assembled and disassembled, sterilized and/or repaired. The apparatus may also provide a sealing engagement with a protective garment and include one or more usage trackers to monitor and track the usage of one or more of the components of the apparatus.

According to one aspect, a protective eyewear apparatus according to claim 1 is provided.

The brace may comprise a forehead brace adapted to contact the forehead of a wearer.

The brace may comprise a nose brace adapted to fit over a nose of a wearer.

The brace may comprise a forehead brace and a nose brace, the forehead brace and the nose brace adapted to position the lens a distance from a wearer's eye.

The brace may be a compressible material, a rigid material or a semi-rigid material.

The body may comprise a nose cup sized and shaped to fit over the nose of a wearer, the lens defining a nose gap sized and shape to receive the nose cup.

The body may be curved about the inner surface and adapted to substantially fit a wearer's face, the lens and the cover plate being similarly curved.

The lens may be nearly 100% transparent or may be shaded to a degree of opacity.

The lens may be disposable.

The protective eyewear may further comprise a cover for the third receptacle.

The protective eyewear apparatus may further comprise at least one usage tracker disposed in one of the first, second, or third receptacles.

The usage tracker may be a microchip.

The usage tracker may comprise a radio frequency identification chip.

The engagement of the lens with the body and the cover plate with the lens and body may form a substantially air-tight seal.

The protective eyewear apparatus may further comprise a garment having at least one edge, the at least one edge disposed in the perimeter groove of the body and secured to the body by the cover plate, the engagement of the cover plate with the edge of the garment and the body forming a substantially air-tight seal.

According to another aspect, a protective garment according to claim 13 is provided.

### BRIEF DESCRIPTION OF THE DRAWINGS

The above and other objects of the present invention will become more readily apparent from the following detailed description taken in connection with the accompanying drawings.
Fig. 1 is a top-view of a lens according to an embodiment of the invention.
Fig. 2 is a front-view of a lens according to an embodiment of the invention.
Fig. 3 is a side-view of a lens according to an embodiment of the invention.
Fig. 4 is a front-view of a cover plate according to an embodiment of the invention.
Fig. 5 is a rear-view of a cover plate according to an embodiment of the invention.
Fig. 6 is a side-view of a cover plate according to an embodiment of the invention.
Fig. 7 is a top-view of a cover plate according to an embodiment of the invention.
Fig. 8 is a front-view of an apparatus body according to an embodiment of the invention.
Fig. 9 is a rear-view of an apparatus body according to an embodiment of the invention.
Fig. 10 is a top-view of an apparatus body according to an embodiment of the invention.
Fig. 11 is a side-view of an apparatus body according to an embodiment of the invention.
Fig. 12 is a front-view of an apparatus body with a lens according to an embodiment of the invention.
Fig. 13 is a top-view of an apparatus body with a lens according to an embodiment of the invention.
Fig. 14 is a side-view of an apparatus body with a lens according to an embodiment of the invention.
Fig. 15 is a front-view of an eyewear apparatus according to an embodiment of the invention.
Fig. 16 is a side-view of an eyewear apparatus according to an embodiment of the invention.
Fig. 17 is a top-view of an eyewear apparatus according to an embodiment of the invention.
Fig. 18 is a rear-view of an eyewear apparatus according to an embodiment of the invention.
Fig. 19 is a front-view of an eyewear apparatus integrated with a protective garment according to an embodiment of the invention.
Fig. 20 is a side-view of an eyewear apparatus integrated with a garment according to an embodiment of the invention.

### DETAILED DESCRIPTION

Embodiments of the present invention include a protective eyewear apparatus assembled from a series of components sized and shaped to interconnect to form a sealing engagement between the components and a safety garment, such as a hood or suit. The components and configuration of the eyewear assembly are particularly advantageous for use in a controlled environment, such as a pharmaceutical laboratory or any environment in which individuals should be protected from contaminants and in which the controlled environments should be protected from contaminants carried by an individual. Embodiments of the present invention may be assembled from a frame, including a body and a cover plate, and an interchangeable, or removeable lens. Each component, i.e., the body, the cover plate, and the lens may further include a usage tracker configured to track and monitor the lifespan and other details of the components.

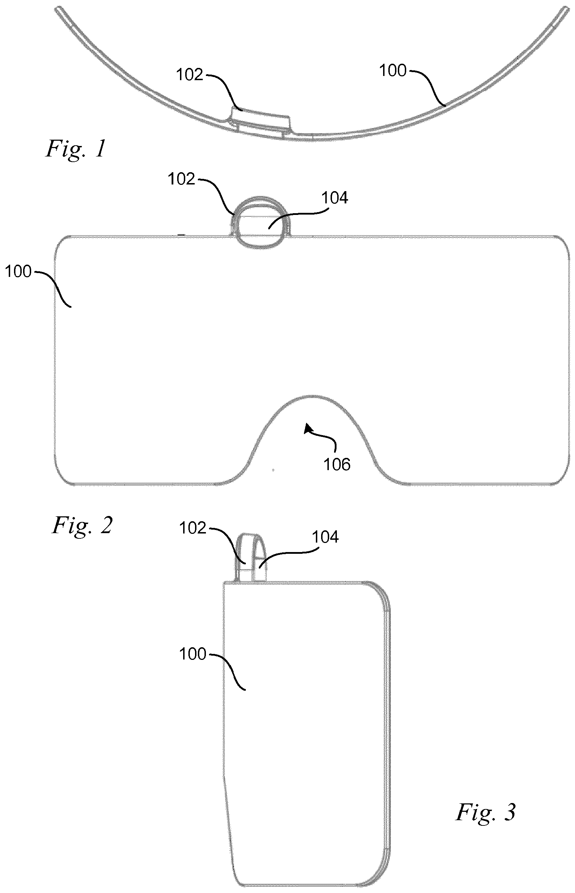

Turning now to Figs. 1-3, a removeable lens 100 is depicted according to one embodiment of the invention. Fig. 1 is a top-view, Fig. 2 is a front-view, and Fig. 3 is a side view of the interchangeable lens 100. The lens 100 may be substantially rectangular with a defined nose gap 106. The lens 100 may also include a usage tracker receptacle 102 adapted to receive a usage tracker. The usage tracker receptacle 102, according to one embodiment, may be located, defined, or formed along a top edge of the lens 100. The usage tracker receptacle 102 may be substantially circular and may include a retainer bar 104 to support and help retain the usage tracker in place. The lens 100 and the receptacle 102 may be formed from a unitary body, made from a mold, 3D printer, or other manufacturing process known in the art. Alternatively, the lens 100 and usage tracker receptacle 102 may be separately formed and joined together using any number of techniques known in the art, including, but not limited to, adhesives, fasteners, plastic or other welds, or the like.

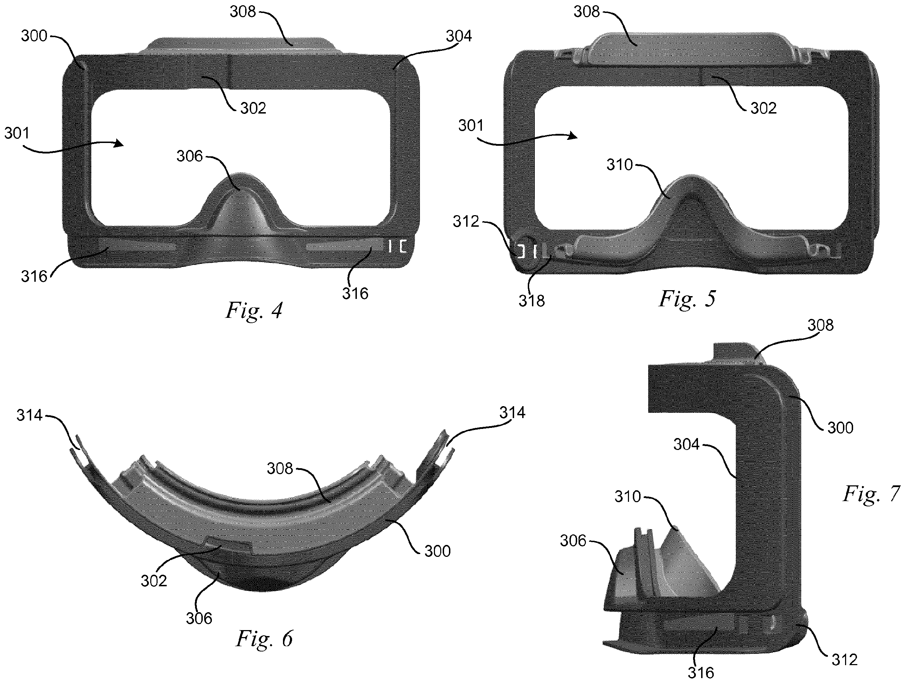

The lens 100 may be formed from a plastic or other polymeric material. The lens may be translucent or semi-translucent depending on the application for which the apparatus in intended. The lens 100 may be nearly 100% transparent or it may be shaded or tinted to various degrees of opacity to block or otherwise impede certain types of light, such as ultraviolet or other light spectra. The lens 100 may further be formed from, or include as a coating or film, a scratch resistant material, an anti-fogging material, or other lens treatments. The lens 100 may be appropriately sized and shaped to conform to an apparatus body 300, such as that shown in Figs. 4-7, to form an air-tight seal between the lens 100 and the body 300.

Figs. 4-7 depict an eyewear apparatus body 300 according to an embodiment of the invention. Fig. 4 is a front-view, Fig. 5 is a rear-view, Fig. 6 is a top-view, and Fig. 7 is a side-view of the body 300. The body 300 may include an inner surface and an outer surface. The body may be substantially rectangular shaped and define a window 301 into or over which the removable lens 100 may be disposed. The body 300 may further form a nose cup 306 generally adapted and shaped to cover the wearer's nose. The nose cup 306 may be appropriately sized and shaped to receive the nose gap of the lens, described above, including a lens groove including a nose cup groove 304 extending along the nose cup 306 to the outer edges of the window 301 and horizontal grooves 304 on the sides of the window 301. The outer surface of the body 300 may further define an indentation 302 to accommodate the usage tracker receptacle 102 of the lens. The body 300 may be formed from a plastic or other polymeric material. The body 300 may also be formed from a mold, 3-D printer or other manufacturing process known in the art.

The inner surface of the body 300 may include one or more braces or supports extending inwardly from the body towards where a wearer's face may be located when the eyewear apparatus is worn. For example, a forehead brace 308 may be attached to the inner surface of the body 300 shaped and positioned to abut an individual's forehead when the apparatus is worn. Similarly, a nose brace 310 may be attached to the inner surface of the body 300. The nose brace 310 may be generally sized and shaped to fit over the nose of an individual wearer. The forehead brace 308 and the nose brace 310 may further be sized and shaped to create an appropriate distance from the individual wearer's eyes and face to the lens 100, when installed, allowing for proper focus and air-movement to reduce fogging.

The forehead brace 308 and the nose brace 310 may be substantially or semi-rigid and formed from a mold, 3-D printer or other manufacturing process known in the art. Alternatively, the braces 308, 310 may be made from a flexible material such as a foam, soft plastic, or other compressible material. Each brace may be attached or affixed to the body by a number of methods, including adhesives, snap-fit, friction fit or the like. For example, the body 300 may include attachment holes 316 and/or tabs 318 through and under which a portion of the nose brace 310 may be inserted to form a secure and tight connection. Alternatively, the entire body 300, including the one or more braces 308, 310 may be formed, molded, 3-D printed or otherwise manufactured as a single piece.

The body 300 may also contain or define a usage tracker receptacle 312 sized and shaped to accommodate a usage tracker. The usage tracker receptacle 312 may be located where it will not obstruct the vision or feel of the body 300 when worn by an individual, such as the lower corner of the inner surface of the body 300. The body 300 may also include or define a perimeter groove 314 about the outer edges of the body 300. The perimeter groove 314 may function as a receiver for a cover plate 400, as described below, that secures the lens to the body 300. As further described below, the perimeter groove 314 may also serve as a insertion area for a garment, such as a hood, whereby the cover plate when attached to the body 300 may form a securing fit clamping the garment in the perimeter groove 314 and sealing the inside of the garment.

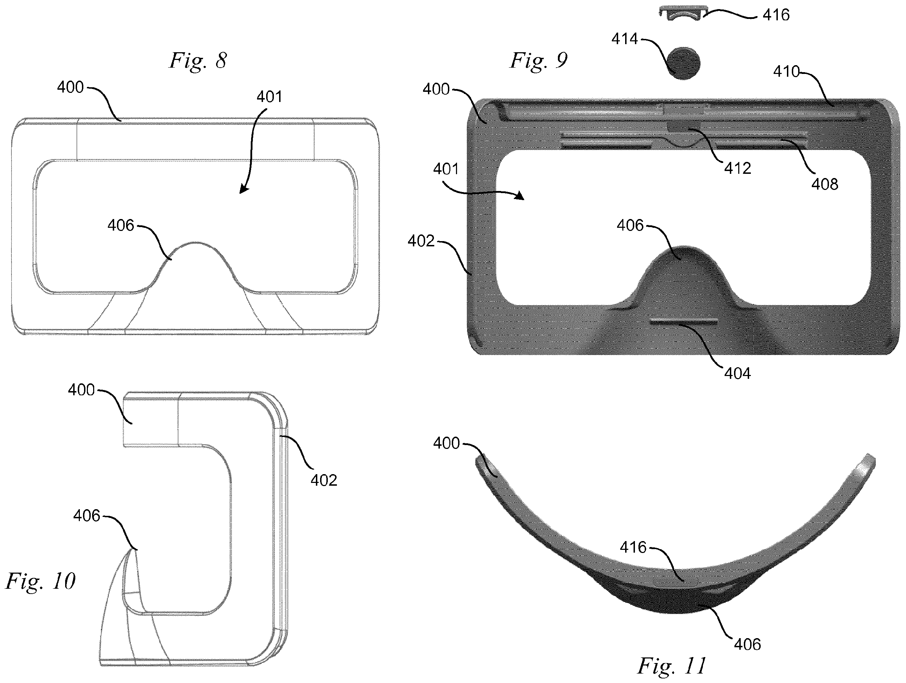

Figs. 8-11 depict a cover plate 400 according to an embodiment of the invention. Fig. 8 is a front view, Fig. 9 is a rear view, Fig. 10 is a side-view and Fig. 11 is a top-view of the cover plate 400. The cover plate 400 may be substantially rectangular and define a nose cover 406 and a cover window 401. The cover window 401 may be sized and shaped to be smaller than the removable lens 100, such that the entire perimeter of the lens 100 is covered and secured by the cover plate 400. The cover plate 400 may be formed, molded, 3-D printed, or otherwise manufactured according to known methods. The cover plate 400 may include a ridge 402 about a portion or all of the perimeter. The ridge 402 may be substantially perpendicular from the cover plate 400 surface and be sized and shaped to form a fitting engagement with the perimeter groove 314 of the body 300. The cover plate 400 may further include additional securing fixtures on the inside surface, such as a nose ridge 404, and forehead ridges 408, 410, to form a more secure, friction fit between the cover plate 400 and the body 300 and the lens 100. The cover plate 400 may be rounded or formed about an arc to give the cover plate 400 a shape matching that of the lens 100 and the body 300.

The cover plate 400 may include or define a usage tracker receptacle 412. A usage tracker 414, as described in further detail herein, may be inserted into the usage tracker receptacle 412 and secured with a clip 416. The clip 416 may be sized and shaped to form a friction or compression fit within or over the usage tracker receptacle 412. Alternatively, the clip 416 may be secured to the cover plate using any number of securing devices, such as a fastener, adhesive or the like.

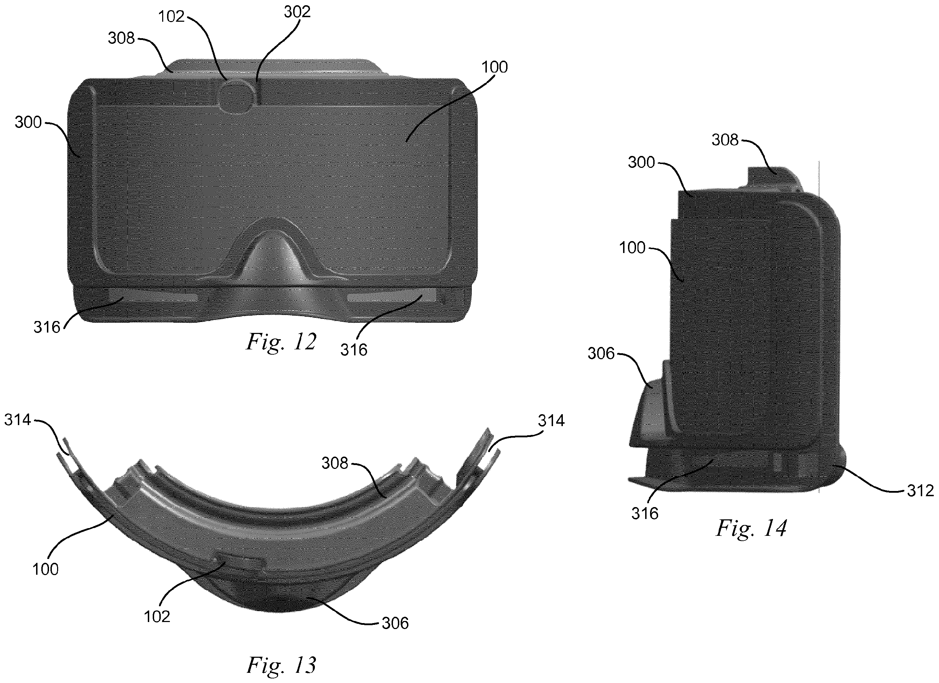

Figs. 12-14 depict an assembly of the lens 100 and the body 300 according to an embodiment of the invention. Fig. 12 is a front-view, Fig. 13 is a top-view and Fig. 14 is a side-view of the lens/body assembly. The lens 100 may be inserted or slid into the horizontal grooves 304 and nose groove 305 over the body window 301 to form a sealing engagement with the body 300. The lens usage tracker receptacle 102, shown with a usage tracker installed, may align with the indentation 302 in the body 300 to reduce the profile thickness of the lens/body assembly.

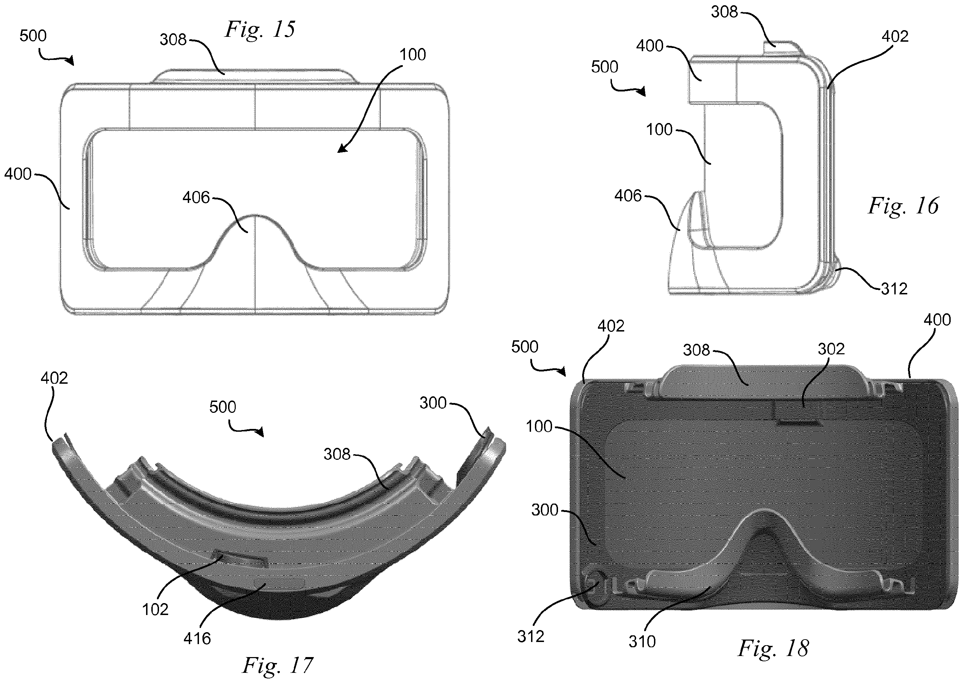

Figs. 15-18 depict a eyewear assembly 500 according to an embodiment of the invention. Fig. 15 is a front-view, Fig. 16 is a side-view, Fig. 17 is a top-view and Fig. 18 is a rear-view of the eyewear assembly 500. After the removable lens 100 is in place, the cover plate 400 may be engaged over the lens 100 into a fitting engagement with the perimeter groove 314 of the body 300. In one embodiment, the cover plate 400 may be secured over an edge of the garment fabric or material. The edge may be inserted into the perimeter groove 314, whereby the cover plate 400 clamps or otherwise secures the garment to the eyewear assembly 500.

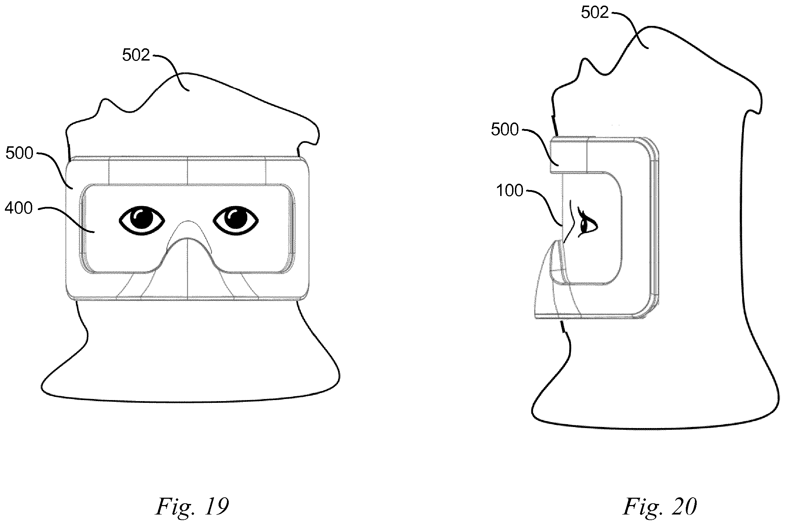

Figs. 19 and 20 depict an eyewear assembly 500 integrated with a garment 502. Fig. 19 is a front-view, Fig. 20 is a side-view of assembly/garment integration. The eyewear assembly 500, when integrated with a hood garment 502, provides a sealed environment inside the garment with adequate eyewear to allow the individual wide angles of viewing, and a sealed environment inside of the garment.

Usage trackers may be implemented with one or more of the components of the eyewear assembly 500. A usage tracker may be in the form of an electronic microchip, or other device sized and shaped according to the usage tracker receptacles described herein. The usage tracker may track aspects of the individual lifespan of each component. According to one embodiment, the usage tracker may be or include a radio frequency identification (RFID) chip that is detectable, readable and contains identifying information related to the eyewear assembly 500, and its usage. For example, a government standard or other best-practice requirement may dictate that certain components only be used in a controlled environment for a limited period of time or number of sterilizations. In such cases, the usage tracker may be scanned upon insertion into the eyewear component, recording the assembly's date, time, and other pertinent information related to its first use. Each time the eyewear device undergoes a sterilization or cleaning process, the usage tracker may be scanned and subsequent usage information may be recorded. Usage information may include, without limitation, identifying information, time and date stamps, number of sterilization cycles, or other periodic information. When the eyewear assembly reaches a maximum number of cycles, or other expiration factor, the scanning device may notify the user that the assembly should be retired, recycled or otherwise disposed of.

Embodiments of the eyewear assembly may be designed for reuse and/or replacement. For example, the lens 100, the body 300, and the cover plate 400 may be manufactured such that the components may be sterilized in an autoclave for a number of life cycles. For example, the components may be made from materials that are suitable for sterilization in an autoclave at about 121° C for a given number of cycles, for example 15 to 30 cycles. Further, the usage trackers may be designed to withstand such sterilization and monitor and track the number of sterilization cycles. Alternatively, one or more components may be designed to be disposable and replaceable. For example, the lens 100 may be manufactured to a lesser quality, reducing the cost of the lens 100, such that the individual may simply discard the lens 100 after a given usage cycle. The body 300 and the cover plate 400 may still be high quality materials able to withstand sterilization and re-use. As such, a durable, efficient, and effective eyewear apparatus with an integrated safety garment provides several technical advantages of traditional safety equipment.

References to items in the singular should be understood to include items in the plural, and vice versa, unless explicitly stated otherwise or clear from the text. Grammatical conjunctions are intended to express any and all disjunctive and conjunctive combinations of conjoined clauses, sentences, words, and the like, unless otherwise stated or clear from the context. Thus, the term "or" should generally be understood to mean "and/or" and so forth.

Recitation of ranges of values herein are not intended to be limiting, referring instead individually to any and all values falling within the range, unless otherwise indicated herein, and each separate value within such a range is incorporated into the specification as if it were individually recited herein. The words "about," "approximately," "substantially," or the like, when accompanying a numerical value or direction are to be construed as indicating a deviation as would be appreciated by one of ordinary skill in the art to operate satisfactorily for an intended purpose. Ranges of values and/or numeric values are provided herein as examples only, and do not constitute a limitation on the scope of the described embodiments. The use of any and all examples, or exemplary language ("e.g.," "such as," or the like) provided herein, is intended merely to better illuminate the embodiments.

In the preceding description, it is understood that terms such as "first," "second," "third," "above," "below," and the like, are words of convenience and are not to be construed as limiting terms unless expressly state otherwise.

It will be appreciated that the methods and systems described above are set forth by way of example and not of limitation. Numerous variations, additions, omissions, and other modifications will be apparent to one of ordinary skill in the art, within the scope of the appended claims. In addition, the order or presentation of method steps in the description and drawings above is not intended to require this order of performing the recited steps unless a particular order is expressly required or otherwise clear from the context.

## Claims

1. A protective eyewear apparatus (500) comprising:
a body (300) having an inner and outer surface, the body defining a perimeter groove (314) about an outer edge of the body (300) and a lens groove (304), the body further comprising a first receptacle (312) adapted to receive a first usage tracker configured for tracking at least one aspect of the lifespan of the body (300);
a brace (308, 310) affixed to the inner surface of the body;
a removable lens (100) disposed in the lens groove (304) and adjacent the outer surface of the body, the lens comprising a second receptacle (102) adapted to receive a second usage tracker configured for tracking at least one aspect of the lifespan of the removable lens (100);
a cover plate (400) sized and shaped to engage the perimeter groove (314) of the body and form a sealing engagement over the removable lens, the cover plate comprising a third receptacle (412) adapted to receive a third usage tracker configured for tracking at least one aspect of the lifespan of the cover plate (400).

2. The protective eyewear apparatus of claim 1 wherein the brace comprises a forehead brace (308) adapted to contact the forehead of a wearer.

3. The protective eyewear apparatus of claim 1 or claim 2 wherein the brace comprises a nose brace (310) adapted to fit over a nose of a wearer.

4. The protective eyewear apparatus of any of the preceding claims wherein the brace comprises a forehead brace (308) and a nose brace (310), the forehead brace and the nose brace adapted to position the lens a distance from a wearer's eye.

5. The protective eyewear apparatus of any of the preceding claims wherein the brace (308, 310) is a compressible material, or
wherein the brace is a rigid material, or
wherein the brace is a semi-rigid material.

6. The protective eyewear apparatus of any of the preceding claims wherein the body comprises a nose cup (306) sized and shaped to fit over the nose of a wearer, the lens (100) defining a nose gap (106) sized and shape to receive the nose cup.

7. The protective eyewear apparatus of any of the preceding claims wherein the body (300) is curved about the inner surface and adapted to fit a wearer's face, the lens (100) and the cover plate (400) being similarly curved.

8. The protective eyewear apparatus of any of the preceding claims wherein the lens (100) is 100% transparent, or
wherein the lens (100) is shaded.

9. The protective eyewear apparatus of any of the preceding claims wherein the lens (100) is configured to be disposable.

10. The protective eyewear apparatus of any of the preceding claims further comprising a cover for the third receptacle (412).

11. The protective eyewear apparatus of any of the preceding claims further comprising at least one usage tracker disposed in one of the first, second, or third receptacles, each usage tracker being configured for tracking at least one aspect of the individual lifespan of the body (300), removable lens (100) or cover plate (400) respectively and, optionally,
wherein the usage tracker is a microchip, and/or
wherein the usage tracker comprises a radio frequency identification chip.

12. The protective eyewear apparatus of any of the preceding claims wherein the engagement of the lens (100) with the body (300) and the cover plate (400) with the lens (100) and body (300) is configured to form an air-tight seal.

13. A protective garment comprising:
a hood or body-suit garment (502) having at least one edge; and
a protective eyewear apparatus (500) according any of claims 1 to 12,
wherein the at least one edge of the hood or body-suit garment (502) is disposed in the perimeter groove (314), and the cover plate (400) is sized and shaped to form a sealing engagement over the at least one edge of the hood garment (502) and the removable lens (100).

## Patentansprüche

1. Schutzbrilleneinrichtung (500), umfassend:
einen Körper (300), der eine Innen- und Außenfläche aufweist, wobei der Körper eine Umfangsnut (314) um einen Außenrand des Körpers (300) herum und eine Brillenglasnut (304) definiert, wobei der Körper weiter eine erste Aufnahme (312) umfasst, die so angepasst ist, dass sie einen ersten Verwendungs-Tracker aufnimmt, der dafür konfiguriert ist, mindestens einen Aspekt der Lebensdauer des Körpers (300) zu verfolgen;
einen Bügel (308, 310), der an der Innenfläche des Körpers angebracht ist;
ein herausnehmbares Brillenglas (100), das in der Brillenglasnut (304) und an die Außenfläche des Körpers angrenzend angeordnet ist, wobei das Brillenglas eine zweite Aufnahme (102) umfasst, die so angepasst ist, dass sie einen zweiten Verwendungs-Tracker aufnimmt, der dafür konfiguriert ist, mindestens einen Aspekt der Lebensdauer des herausnehmbaren Brillenglases (100) zu verfolgen;
eine Abdeckplatte (400), die so bemessen und geformt ist, dass sie in die Umfangsnut (314) des Körpers eingreift und einen dichtenden Eingriff über dem herausnehmbaren Brillenglas bildet, wobei die Abdeckplatte eine dritte Aufnahme (412) umfasst, die so angepasst ist, dass sie einen dritten Verwendungs-Tracker aufnimmt, der dafür konfiguriert ist, mindestens einen Aspekt der Lebensdauer der Abdeckplatte (400) zu verfolgen.

2. Schutzbrilleneinrichtung nach Anspruch 1, wobei der Bügel einen Stirnbügel (308) umfasst, der so angepasst ist, dass er die Stirn eines Trägers berührt.

3. Schutzbrilleneinrichtung nach Anspruch 1 oder Anspruch 2, wobei der Bügel einen Nasenbügel (310) umfasst, der so angepasst ist, dass er über eine Nase eines Trägers passt.

4. Schutzbrilleneinrichtung nach einem der vorstehenden Ansprüche, wobei der Bügel einen Stirnbügel (308) und einen Nasenbügel (310) umfasst, wobei der Stirnbügel und der Nasenbügel so angepasst sind, dass sie das Brillenglas in einem Abstand vom Auge eines Trägers positionieren.

5. Schutzbrilleneinrichtung nach einem der vorstehenden Ansprüche, wobei der Bügel (308, 310) aus einem komprimierbaren Material ist, oder
wobei der Bügel aus einem starren Material ist, oder
wobei der Bügel aus einem halbstarren Material ist.

6. Schutzbrilleneinrichtung nach einem der vorstehenden Ansprüche, wobei der Körper eine Nasenschale (306) umfasst, die so bemessen und geformt ist, dass sie über die Nase eines Trägers passt, wobei das Brillenglas (100) einen Nasenspalt (106) definiert, der so bemessen und geformt ist, dass er die Nasenschale aufnimmt.

7. Schutzbrilleneinrichtung nach einem der vorstehenden Ansprüche, wobei der Körper (300) um die Innenfläche herum gekrümmt und so angepasst ist, dass er zum Gesicht eines Trägers passt, wobei das Brillenglas (100) und die Abdeckplatte (400) ähnlich gekrümmt sind.

8. Schutzbrilleneinrichtung nach einem der vorstehenden Ansprüche, wobei das Brillenglas (100) 100 % transparent ist, oder
wobei das Brillenglas (100) getönt ist.

9. Schutzbrilleneinrichtung nach einem der vorstehenden Ansprüche, wobei das Brillenglas (100) so konfiguriert ist, dass es weggeworfen werden kann.

10. Schutzbrilleneinrichtung nach einem der vorstehenden Ansprüche, die weiter eine Abdeckung für die dritte Aufnahme (412) umfasst.

11. Schutzbrilleneinrichtung nach einem der vorstehenden Ansprüche, die weiter mindestens einen Verwendungs-Tracker umfasst, der in einer der ersten, der zweiten oder der dritten Aufnahme angeordnet ist, wobei jeder Verwendungs-Tracker dafür konfiguriert ist, mindestens einen Aspekt der einzelnen Lebensdauer des Körpers (300), des herausnehmbaren Brillenglases (100) bzw. der Abdeckplatte (400) zu verfolgen, und gegebenenfalls
wobei der Verwendungs-Tracker ein Mikrochip ist, und/oder
wobei der Verwendungs-Tracker einen Funkfrequenz-Identifikationschip umfasst.

12. Schutzbrilleneinrichtung nach einem der vorstehenden Ansprüche, wobei der Eingriff des Brillenglases (100) in den Körper (300) und der Abdeckplatte (400) in das Brillenglas (100) und den Körper (300) so konfiguriert ist, dass er eine luftdichte Dichtung bildet.

13. Schutzkleidung, umfassend:
eine Hauben- oder Overallkleidung (502), die mindestens einen Rand aufweist; und
eine Schutzbrilleneinrichtung (500) nach einem der Ansprüche 1 bis 12,
wobei der mindestens eine Rand der Hauben- oder Overallkleidung (502) in der Umfangsnut (314) angeordnet ist und die Abdeckplatte (400) so bemessen und geformt ist, dass sie einen dichtenden Eingriff über dem mindestens einen Rand der Haubenkleidung (502) und dem herausnehmbaren Brillenglas (100) bildet.

## Revendications

1. Un appareil de protection oculaire (500) comprenant :
un corps (300) ayant une surface intérieure et extérieure, le corps définissant une rainure périphérique (314) autour d'un bord extérieur du corps (300) et une rainure de lentille (304), le corps comprenant en outre un premier réceptacle (312) adapté pour recevoir un premier traceur d'utilisation configuré pour suivre au moins un aspect de la durée de vie du corps (300) ;
une entretoise (308, 310) fixée à la surface intérieure du corps ;
une lentille amovible (100) disposée dans la rainure de lentille (304) et adjacente à la surface extérieure du corps, la lentille comprenant un second réceptacle (102) adapté pour recevoir un second traceur d'utilisation configuré pour suivre au moins un aspect de la durée de vie de la lentille amovible (100) ;
une plaque de recouvrement (400) dimensionnée et formée pour s'engager dans la rainure périphérique (314) du corps et former un engagement étanche sur la lentille amovible, la plaque de recouvrement comprenant un troisième réceptacle (412) adapté pour recevoir un troisième traceur d'utilisation configuré pour suivre au moins un aspect de la durée de vie de la plaque de recouvrement (400).

2. L'appareil de protection oculaire de la revendication 1, dans lequel l'entretoise comprend une entretoise frontale (308) adaptée pour entrer en contact avec le front d'un porteur.

3. L'appareil de protection oculaire de la revendication 1 ou de la revendication 2, dans lequel l'entretoise comprend une entretoise nasale (310) adaptée pour s'adapter au nez d'un porteur.

4. L'appareil de protection oculaire de l'une des revendications précédentes, dans lequel l'entretoise comprend une entretoise frontale (308) et une entretoise nasale (310), l'entretoise frontale et l'entretoise nasale étant adaptées pour positionner la lentille à une certaine distance de l'oeil de l'utilisateur.

5. L'appareil de protection oculaire de l'une quelconque des revendications précédentes, dans lequel l'entretoise (308, 310) est un matériau compressible, ou
dans lequel l'entretoise est un matériau rigide, ou
dans lequel l'entretoise est un matériau semi-rigide.

6. L'appareil de protection oculaire de l'une des revendications précédentes, dans lequel le corps comprend un masque nasal (306) dimensionné et formé pour s'adapter au nez d'un porteur, la lentille (100) définissant un espace nasal (106) dimensionné et formé pour recevoir le masque nasal.

7. L'appareil de protection oculaire de l'une des revendications précédentes, dans lequel le corps (300) est incurvé sur la surface intérieure et adapté au visage du porteur, la lentille (100) et la plaque de recouvrement (400) étant incurvées de la même manière.

8. L'appareil de protection oculaire de l'une des revendications précédentes dans lequel la lentille (100) est 100% transparente, ou dans lequel la lentille (100) est ombrée.

9. L'appareil de protection oculaire de l'une des revendications précédentes dans lequel la lentille (100) est configurée pour être jetable.

10. L'appareil de protection oculaire de l'une des revendications précédentes, comprenant en outre un couvercle pour le troisième réceptacle (412).

11. L'appareil de protection oculaire de l'une des revendications précédentes, comprenant en outre au moins un traceur d'utilisation disposé dans l'un des premier, deuxième ou troisième réceptacles, chaque traceur d'utilisation étant configuré pour suivre au moins un aspect de la durée de vie individuelle du corps (300), de la lentille amovible (100) ou de la plaque de recouvrement (400) respectivement et, éventuellement,
dans lequel le traceur d'utilisation est une puce électronique, et/ou
dans lequel le traceur d'utilisation comprend une puce d'identification par radiofréquence.

12. L'appareil de protection oculaire de l'une des revendications précédentes, dans lequel l'engagement de la lentille (100) avec le corps (300) et de la plaque de recouvrement (400) avec la lentille (100) et le corps (300) est configuré pour former un joint étanche à l'air.

13. Un vêtement de protection comprenant :
une cagoule ou un vêtement de corps (502) ayant au moins un bord ; et
un appareil de protection oculaire (500) selon l'une des revendications 1 à 12,
dans lequel au moins un bord de la cagoule ou du vêtement (502) est disposé dans la rainure périphérique (314), et la plaque de recouvrement (400) est dimensionnée et formée pour former un engagement étanche sur au moins un bord de la cagoule (502) et de la lentille amovible (100).
